# EUROPEAN PATENT APPLICATION

(11) **EP 1 974 751 A1**
(43) Date of publication of application: **01.10.2008**
(21) Application number: 07006159.3
(22) Date of filing: 26.03.2007
(51) Int. Cl.: A61K 47/18, A61K 47/40, A61K 47/48, A61K 9/16, A61K 9/68, A61K 9/20

(54) **Formulations for non-steroidal anti-inflammatory drugs**

(71) Applicant: The Jordanian Pharmaceutical Manufacturing Co., 11710 Naor (JO)
(72) Inventor: Badwan, Adnan Dr., 11185 Amman (JO); Daraghmeh, Nidal Hamdan Dr., 11947 Amman (JO); Omari, Mahmoud Mustafa H. Al Dr., 11821 Amman (JO)
(74) Representative: Winkler, Andreas Fritz Ernst

(57) **Abstract**

The present invention provides a composition comprising (i) a non-steroidal anti-inflammatory drug (NSAID) selected from naphthalene and benzene acetic acid derivatives or pharmaceutically acceptable salts thereof and (ii) a mixture of at least one alkyl amine and of at least one cyclodextrin or derivative thereof.

## Description

### FIELD OF THE INVENTION

This invention relates to pharmaceutical dosage forms containing a non-steroidal anti-inflammatory drug (NSAID).

### BACKGROUND OF THE INVENTION

It is known that a number of NSAIDs from the group of naphthalene or benzene acetic acid derivatives such as naproxen, diclofenac, mefenamic acid, flufenamic acid, ibuprofen, ketoprofen and flurbiprofen, have a chemesthetic effect (irritant effect) on the oral cavity, throat and pharynx.

This makes pharmaceutical oral dosage forms for oral use containing naproxen, diclofenac, mefenamic acid, flufenamic acid, ibuprofen, ketoprofen and flurbiprofen or enantiomers and/or salts thereof irritant and unpleasant, when such pharmaceutical forms are in the form of, for example, partially swallowable or chewable tablets, orosoluble tablets, granules and powders to be suspended or dissolved before administration, mouthwashes, sprays, cough drops, lozenges, syrups, drops, oral gels and the like.

Many investigations have been carried out so far to improve the patient tolerability of oral forms based on NSAIDs such as ibuprofen, naproxen and flurbiprofen.

However, the ingredients that have been found to be capable of reducing the irritant stimulus on the oral cavity, throat and pharynx have also been found to give to the oral forms taste properties, such as bitterness and lye-taste, that resulted to be unacceptable.

Mainly, NSAIDs suffer from low solubility in water and have unpleasant irritant taste when administered orally. Therefore, there is still a great need for ingredients capable of eliminating the unacceptable tastes of NSAIDs and of producing a deliverable oral form based on NSAIDs.

Surprisingly it has now been found that this goal is achieved when a mixture of alkyl amine and cyclodextrin is added to a dosage form comprising a NSAID selected from the group consisting of naphthalene and benzene acetic acid derivatives, such as naproxen, diclofenac, mefenamic acid, flufenamic acid, ibuprofen, ketoprofen and flurbiprofen or enantiomers, or pharmaceutically acceptable salts thereof.

### SUMMARY OF THE INVENTION

Therefore, in a first aspect the present invention relates to an pharmaceutical oral dosage form comprising (i at least one non-steroidal anti-inflammatory drug (NSAID) selected from the group consisting of naphthalene and benzene acetic acid derivatives or pharmaceutically acceptable salts thereof and (ii) a mixture of at least one alkyl amine, preferably tromethamine, and at least one cyclodextrin or derivative thereof, preferably ß-cyclodextrin or derivative thereof.

NSAIDs suffer from low water solubility that often hampers their application. To enhance their solubility, they are normally converted to alkali salts such as sodium or potassium salts or converted to alkylammonium salts, i.e. lysine or tromethamine. However, sometimes even the produced salts have limited solubility to achieve the target concentration, mainly in oral and parenteral solutions. A conventional salt formation is therefore often inadequate (e.g. stability, process).

Therefore, a second aspect of the present invention relates to the preparation of complexes with a cyclodextrin (CD), and particularly with β-cyclodextrin, with the purpose of increasing water solubility.

The parent cyclodextrins α-, β-, γ-cyclodextrins and their derivatives are widely used to improve the solubility of water insoluble compounds through inclusion complexation (dissolution and bioavailability improvements). Also they are used to reduce irritation, as a taste-masking agent, to enhance the thermal stability, to reduce volatility, and to resist oxidation, hydrolysis and degradation of compounds in solution.

The parent cyclodextrins vary in their water solubility. While γ-cyclodextrin is the most water soluble (23.2 g/100 ml at 25°C), β-cyclodextrin is the least soluble (1.8 g/100 ml at 25°C). Solubility of β-cyclodextrin can be enhanced by preparing different derivatives of this polymer such as hydroxypropyl-β-cyclodextrins and sulfobutylether-β-cyclodextrins. These polymers efficiency in solublization of the water insoluble compounds is directly proportional to the solubility of the polymers in the system.

Among all CDs used, β-cyclodextrin is the most utilized in pharmaceutical investigations due to its abundance and cavity diameter. It is sparingly soluble in water. This limited aqueous solubility is attributed to a relatively high crystal lattice energy and the existence of intramolecular hydrogen bonding between the hydroxyl groups of β-cyclodextrin.

Another aspect of this invention is a general method for preparation of a NSAID alkyl amine/cyclodextrin complex by removal of water from the highly supersaturated solution of the three components in water.

### DESCRIPTION OF THE DRAWINGS

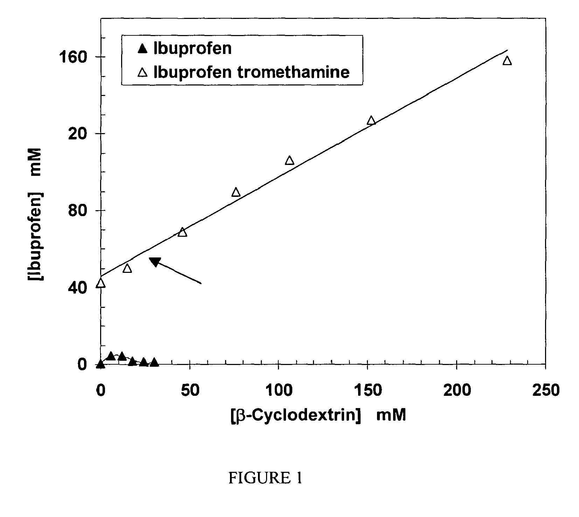

**Figure 1** shows the phase solubility diagrams obtained for ibuprofen/β-cyclodextrin and ibuprofen tromethamine/β-cyclodextrin in water.

### DETAILED DESCRIPTION OF THE INVENTION

The invention relates to a pharmaceutical dosage form comprising
1 a NSAID selected from naphthalene and benzene acetic acid derivatives such as naproxen, diclofenac, mefenamic acid, flufenamic acid, ibuprofen, ketoprofen and flurbiprofen,
2 an alkyl amine such as tromethamine, and
3 a solublizer and a taste-masking agent such as a cyclodextrin

The preferred "NSAIDs" and "nonsteroidal anti-inflammatory drug" of this invention are naphthalene and benzene acetic acid derivatives, such as naproxen, diclofenac, mefenamic acid, ibuprofen, ketoprofen and flurbiprofen, as racemate mixtures or as pure or enriched enantiomer forms as well as pharmaceutical acceptable salts thereof.

Preferably, the amount of the alkyl amine ranges from 0.2 to 1.5 parts by weight per 1 part by weight of NSAID. More preferably, the amount alkyl amine ranges from 0.4 to 1.2 parts by weight per 1 part by weight of NSAID, even more preferably, from 0.3 to 0.5 parts by weight per 1 part by weight of NSAID.

Preferably the amount of the cyclodextrin or derivative thereof ranges from 0.05 to 10 parts by weight per 1 part by weight of NSAID. More preferably, the amount cyclodextrin ranges from 1 to 10 parts by weight per 1 part by weight of NSAID, even more preferably, from 2 to 5 parts by weight per 1 part by weight of NSAID.

Ibuprofen is one example of these NSAIDs and has the following formula.

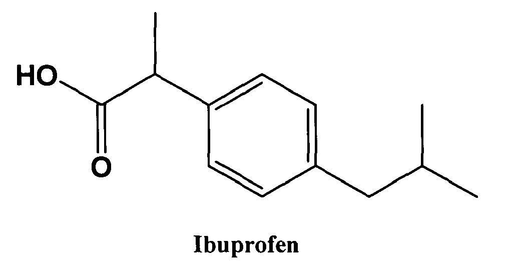

The invention shall now be further described in the following examples, without being limited thereto. The accompanying Figure 1 shows a phase solubility diagram of ibuprofen/β-cyclodextrin and ibuprofen tromethamine salt/β-cyclodextrin systems at 30°C. Arrow represents the optimal solubility of β-cyclodextrin in the absence of ibuprofen tromethamine.

### EXAMPLE 1

### Preparation of Ibuprofen Tromethamine Salt:

Ibuprofen (4.8 mole) was dissolved in ethanol (500 ml) by heating. Tromethamine (4.8 mole) was dissolved in distilled water (500 ml) by heating. Then, the tromethamine aqueous solution was added to the ibuprofen ehanolic solution with vigorous stirring. The precipitate was filtered and dried at 80°C over night.

### EXAMPLE 2

### Preparation of Ibuprofen Tromethamine/ β-Cyclodextrin Complex:

Quantities of NSAID, alkyl amine and cyclodextrin (5 mole each) were dissolved in sufficient water by stirring or/and sonication to obtain a clear or slightly opalescent solution. The solution obtained was dried by using dehydration methods such as freeze drying, spray drying, vacuum drying at moderate temperature or similar.

### EXAMPLE 3

### Mutual Solubility Enhancement of Ibuprofen Tromethamine/ β-Cyclodextrin in Water:

To illustrate the mutual solubility enhancement for both drug and β-cyclodextrin in distilled water, excess amounts of ibuprofen and β-cyclodextrin (well beyond the optimal solubility of β-cyclodextrin corresponding to 20 mM at 30°C). The samples were mechanically shaken in a thermostatic bath shaker (~ 200 rpm) to attain thermal equilibrium; an aliquot was centrifuged (when necessary) and filtered using a 0.45µm filter (cellulose acetate or cellulose nitrate, Advantec MFS Inc., Duplin, USA). The NSAID assay was conducted using UV/vissible spectrophotometry at about 270 nm. β-cyclodextrin assay was performed by optical rotation (α) measurements on a Polartronic D at 25°C using a 1 dm cell.

Figure 1 shows the phase solubility diagrams obtained for ibuprofen/β-cyclodextrin and ibuprofen tromethamine/β-cyclodextrin in water. The samples were shaken for different time intervals. The corresponding β-cyclodextrin solubility was 230 mM (12 times the optimal solubility of β-cyclodextrin, which indicates significant solubility synergism without supersaturation.

### EXAMPLE 4

### Preparation of Ibuprofen Syrup:

| **Component** | **% Weight (w/v)** |
|---|---|
| Ibuprofen | 2.0 |
| Tromethamine | 1.2 |
| Hydroxypropyl-β-cyclodextrin | 15 |
| Sucrose | 30 |
| Glycerin | 25 |
| Aspartame | 0.5 |
| Flavoring agent | 0.7 |
| Coloring agent | 0.01 |
| Preservative | 0.1 |
| Water | Q.S. |

### EXAMPLE 5

### Preparation of Ibuprofen Soluble Granules (Cold Drink):

| **Component** | **% Weight (w/w)** |
|---|---|
| Ibuprofen | 3.5 |
| Tromethamine | 2.0 |
| β-cyclodextrin | 15 |
| Aspartame | 1.0 |
| Fine Sucrose | 72 |
| Povisone-K30 | 1.2 |
| Coloring agent | 0.03 |
| Flavoring agents | 5.0 |

### EXAMPLE 6

### Preparation of Ibuprofen Soluble Granules (Cold Drink):

| **Component** | **% Weight (w/w)** |
|---|---|
| Ibuprofen | 3.5 |
| Tromethamine | 2.0 |
| β-cyclodextrin | 15 |
| Aspartame | 1.5 |
| Mannitol | 72 |
| Povisone-K30 | 1.2 |
| Coloring agent | 0.03 |
| Flavoring agents | 5.0 |

### EXAMPLE 7

### Preparation of Ibuprofen Soluble Granules (Hot Drink):

| **Component** | **% Weight (w/w)** |
|---|---|
| Ibuprofen | 5.0 |
| Tromethamine | 3.0 |
| β-cyclodextrin | 40 |
| Aspartame | 1.5 |
| Mannitol | 45 |
| Povisone-K30 | 1.5 |
| Coloring agent | 0.002 |
| Flavoring agents | 4.0 |

### EXAMPLE 8

### Preparation of Ibuprofen Oral Dissolve Tablets:

| **Component** | **% Weight (w/w)** |
|---|---|
| Ibuprofen | 8.0 |
| Tromethamine | 5.0 |
| β-cyclodextrin | 50 |
| Pharmaburst C | 24 |
| Aspartame | 0.5 |
| Croscarmelose sodium | 5.0 |
| Flavoring agents | 5.0 |
| Magnesium stearate | 2.5 |

### EXAMPLE 9

### Preparation of Ibuprofen Chewing Gum:

| **Component** | **% Weight (w/w)** |
|---|---|
| Ibuprofen Tromethamine | 13 |
| Hydroxypropyl-β-cyclodextrin | 15 |
| Pharmagum | 67 |
| Aspartame | 0.5 |
| Flavoring agent | 2.0 |
| Purified talc | 1.0 |
| Lubricant | 1.5 |

### EXAMPLE 10

### Preparation of Ibuprofen Dispersible Tablet:

| **Component** | **% Weight (w/w)** |
|---|---|
| Ibuprofen Tromethamine | 11 |
| β-cyclodextrin | 50 |
| Aspartame | 1.5 |
| Mannitol | 29.5 |
| Povidone K-30 | 1.5 |
| Flavoring agent | 6.0 |
| Coloring agent | 0.02 |
| Lubricant | 0.5 |

The features disclosed in the foregoing description, claims and drawings may, both separately and in any combination thereof, be material for realising the invention in diverse forms thereof.

## Claims

1. A pharmaceutical dosage form comprising (i) at least one non-steroidal anti-inflammatory drug (NSAID) selected from the group consisting of naphthalene and benzene acetic acid derivatives or pharmaceutically acceptable salts thereof and (ii) a mixture of at least one alkyl amine and at least one cyclodextrin or derivative thereof.

2. The pharmaceutical dosage form according to Claim 1, **characterized in that** the NSAID is selected from the group consisting of naproxen, diclofenac, mefenamic acid, flufenamic acid, ibuprofen, ketoprofen and flurbiprofen or enantiomers thereof.

3. The pharmaceutical dosage form according to Claim 1 or 2, wherein the alkyl amine is tromethamine.

4. The pharmaceutical dosage form according to Claim 1 or 3, wherein the cyclodextrin is β-cyclodextrin or derivatives thereof.

5. The pharmaceutical dosage form according to any of Claims 1 to 4, **characterized in that** it comprises from 0.2 to 1.5 parts by weight of alkyl amine per 1 part by weight of NSAID.

6. A pharmaceutical dosage form according to Claim 5, **characterized in that** it comprises from 0.4 to 1.2 parts by weight of alkyl amine per 1 part by weight of NSAID.

7. A pharmaceutical dosage form according to Claim 5, **characterized in that** it comprises from 0.3 to 0.5 parts by weight of alkyl amine per 1 part by weight of NSAID.

8. A pharmaceutical dosage form according to anyone of Claims 1 to 7, **characterized in that** it comprises from 0.05 to 10 parts by weight of cyclodextrin per 1 part by weight of NSAID.

9. A pharmaceutical dosage form according to Claim 8, **characterized in that** it comprises from 1 to 10 parts by weight of cyclodextrin per 1 part by weight of NSAID.

10. A pharmaceutical dosage form according to Claim 9, **characterized in that** it comprises from 2 to 5 parts by weight of cyclodextrin per 1 part by weight of NSAID.

11. A pharmaceutical dosage form according to Claims 1 to 10, **characterized in that** the dosage form is selected from:
an oral solution, soluble granules, an oral dissolve tablet, a chewing gum, a dispersible tablet, a powder ready for compressing or encapsulating, and a soft gelatin capsule.

12. A pharmaceutical dosage form according to Claims 1 to 10, **characterized in that** the pharmaceutical formulation is selected from:
a topical application system, and a parenteral implant.

13. A pharmaceutical dosage form according to Claims 1 to 12 comprising one or more of other pharmacologically active ingredients.

14. A method for preparation of an NSAID alkylamine/cyclodextrin complex for the use in an pharmaceutical dosage form according to any of claims 1-13 by removal of water from a highly supersaturated solution of the three components in water.

## Amended claims

### Amended claims in accordance with Rule 137(2) EPC.

**1.** A method for preparation of a complex of a non-steroidal anti-inflammatory drug (NSAID), at least one alkyl amine and at least one cyclodextrin or derivative thereof for use in a pharmaceutical dosage form, **characterized in that** a highly supersaturated solution of the three components in water is prepared and the water is removed from said solution.

**2.** The method according to claim 1, **characterized in that** the NSAID is selected from the group consisting of naproxen, diclofenac, mefenamic acid, flufenamic acid, ibuprofen, ketoprofen and flurbiprofen or enantiomers thereof.

**3.** The method according to claim 1 or 2, wherein the alkyl amine is tromethamine.

**4.** The method according to claim 1 or 3, wherein the cyclodextrin is βcyclodextrin.

**5.** The method according to any of claims 1 to 4, **characterized in that** it comprises from 0.2 to 1.5 parts by weight of alkyl amine per 1 part by weight of NSAID.

**6.** The method according to claim 5, **characterized in that** it comprises from 0.4 to 1.2 parts by weight of alkyl amine per 1 part by weight of NSAID.

**7.** The method according to claim 5, **characterized in that** it comprises from 0.3 to 0.5 parts by weight of alkyl amine per 1 part by weight of NSAID.

**8.** The method according to anyone of claims 1 to 7, **characterized in that** it comprises from 0.05 to 10 parts by weight of cyclodextrin per 1 part by weight of NSAID.

**9.** The method according to claim 8, **characterized in that** it comprises from 1 to 10 parts by weight of cyclodextrin per 1 part by weight of NSAID.

**10.** The method according to claim 9, **characterized in that** it comprises from 2 to 5 parts by weight of cyclodextrin per 1 part by weight of NSAID.

**11.** The method according to claims 1 to 10, **characterized in that** the dosage form is selected from: an oral solution, soluble granules, an oral dissolve tablet, a chewing gum, a dispersible tablet, a powder ready for compressing or encapsulating, and a soft gelatin capsule.

**12.** The method according to any of the preceding claim, **characterized in that** the pharmaceutical dosage form further comprises one or more of other pharmacologically active ingredients.
